Europäisches Patentamt

**European Patent Office**

**Office européen des brevets**

(19)

(11) Publication number: **0 137 424**
**A2**

(12) # EUROPEAN PATENT APPLICATION

(21) Application number: **84111575.1**

(22) Date of filing: **27.09.84**

(51) Int. Cl.⁴: **C 07 C 25/13**
**C 07 C 17/32**

(30) Priority: **30.09.83 JP 182450/83**

(43) Date of publication of application:
**17.04.85 Bulletin 85/16**

(84) Designated Contracting States:
**BE CH DE FR GB IT LI NL**

(71) Applicant: **ISHIHARA SANGYO KAISHA LTD.**
**No. 3-22, Edobori 1-chome**
**Nishi-ku Osaka(JP)**

(72) Inventor: **Nasu, Rikuo**
**337, Jofukuji-Higashiiru Ichijodori Kamigyo-ku**
**Kyoto-shi Kyoto(JP)**

(72) Inventor: **Shigehara, Itaru**
**1922-232, Noji-cho**
**Kusatsu-shi Shiga-ken(JP)**

(72) Inventor: **Kawashima, Junichi**
**242, Uda, Minakuchi-cho**
**Koka-gun Shiga-ken(JP)**

(72) Inventor: **Maeda, Masaru**
**1183, Tazuke-cho**
**Hikone-shi Shiga-ken(JP)**

(74) Representative: **Wächtershäuser, Günter, Dr.**
**Tal 29**
**D-8000 München 2(DE)**

(54) Process for producing 2,4-dichlorobenzotrifluoride.

(57) A process for producing 2,4-dichlorobenzotrifluoride, which comprises reacting m-dichlorobenzene with a halogenated methane of the formula $CF_xCl_{4-x}$ where x is 0, 1 or 2, and hydrogen fluoride at a temperature of from 80 to 180°C in the presence of a catalyst of at least one member selected from the group consisting of fluorides of antimony, boron, titanium, tantalum and niobium.

Croydon Printing Company Ltd.

- 1 -

## PROCESS FOR PRODUCING 2,4-DICHLOROBENZOTRIFLUORIDE

The present invention relates to an industrially advantageous process for producing 2,4-dichlorobenzotrifluoride (hereinafter referred to simply as "2,4-DCBTF") which is useful as a raw material for agricultural chemicals, medicines or dyestuffs.

2,4-DCBTF is usually produced by chlorinating 2,4-dichlorotoluene to obtain 2,4-dichlorobenzotrichloride, and then fluorinating the product with hydrogen fluoride. However, the starting material 2,4-dichlorotoluene is expensive, and the process involves a plurality of reaction steps. Under the circumstances, it has been desired to improve the process.

Published European Patent Application No. 8,453 discloses a process for producing aromatic trifluoromethyl compounds including 2,4-DCBTF. However, this application does not disclose any specific process for producing 2,4-DCBTF from m-dichlorobenzene (hereinafter referred to simply as "m-DCB") as the starting material.

Besides, it has been found that when the process of this application was applied to m-DCB i.e. the starting material of the present invention, the desired 2,4-DCBTF is formed only at a level of about 20%. Thus, this process is not industrially satisfactory for the production of 2,4-DCBTF, and an improvement has been still desired.

The present inventors have conducted extensive researches to overcome the difficulty of the process of the above-mentioned European Patent Application No. 8,453, and have found an industrially advantageous process for the production of 2,4-DCBTF, whereby the desired product is obtainable in good yield as high as at least 60%. Thus, the present invention has been accomplished.

Namely, the present invention provides a process for producing 2,4-DCBTF, which comprises reacting m-DCB with a halogenated methane of the formula $CF_xCl_{4-x}$ where x is 0, 1 or 2, and hydrogen fluoride at a temperature within a specific range in the presence of a specific catalyst.

The specific catalyst is at least one member selected from the group consisting of fluorides of antimony, boron, titanium, tantalum and niobium. Further, there may be employed substances which are convertible to such fluorides in the reaction system, such as chlorides, bromides or iodides of the above-mentioned metals. Specifically, there may be mentioned antimony pentafluoride (SbF$_5$), antimony dichlorotrifluoride (SbCl$_2$F$_3$), antimony pentachloride (SbCl$_5$), boron trifluoride (BF$_3$), boron trichloride (BCl$_3$), boron tribromide (BBr$_3$), titanium tetrafluoride (TiF$_4$), titanium tetrachloride (TiCl$_4$), tantalum pentafluoride (TaF$_5$), tantalum pentachloride (TaCl$_5$), tatalum pentabromide (TaBr$_5$), niobium pentafluoride (NbF$_5$), niobium pentachloride (NbCl$_5$) and niobium pentabromide (NbBr$_5$). Among them, at least one member selected from the group consisting of fluorides of antimony, boron, titanium and tantalum, is preferred, including chlorides, bromides and iodides of these metals. From the industrial point of view, at least one member selected from the group consisting of fluorides of antimony, boron and titanium, is further preferred, including chlorides of these metals.

The above-mentioned catalysts may be used alone or in combination as a mixture of at least two different kinds.

The catalyst is usually employed in an amount of from 0.02 to 10 mols, preferably from 0.02 to 1 mol, more preferably from 0.03 to 0.3 mol, per mol of m- DCB as the starting material.

As specific examples of the halogenated methane of the formula CF$_x$Cl$_{4-x}$ where x is 0, 1 or 2, there may be mentioned carbon tetrachloride, trichlorofluoromethane and dichlorodifluoromethane.  Among them, carbon tetrachloride and trichlorofluoromethane are preferred.

From the industrial point of view, carbon tetrachloride is further preferred.

The halogenated methane is usually employed in an amount of from 1 to 10 mols, preferably from 1 to 3 mols,

per mol of m-DCB as the starting material.

Hydrogen fluoride is employed usually in an amount of from 3 to 60 mols, preferably from 9 to 60 mols, more preferably from 9 to 30 mols, per mol of m-DCB as the starting material.

The reaction temperature is from 80 to $180^{\circ}C$,

preferably from 80 to $160^{\circ}C$, more preferably from 100 to

$150^{\circ}C$.

If the reaction conditioins depart substantialy from the above ranges, there will be disadvantages such that the reactivity tends to be poor, or the formation of byproducts tends to increase, whereby the yield will decrease.

The pressure in the closed reactor may be spontaneous pressure under heating in the closed system, which is usually from 5 to 100 $kg/cm^2$.  Further, the air in the closed reactor may be replaced by an inert gas, or the pressure in the reactor may be increased over the level of the above-mentioned spontaneous pressure.  Further, the pressure may be controlled to a predetermined level by appropriately discharging hydrogen chloride gas generated during the reaction.

The reaction is usually completed in from 1 to 20 hours. The obtained reaction mixture is usually subjected to work-up such as extraction or distillation, whereby the desired product is obtainable in good yield.

2,4-DCBTF obtained by the process of the present invention, can be converted to 2,4-dichloro-3,5-dinitrobenzene trifluoride by subjecting it to conventional nitration (see e.g. U.S. Patent 3,586,725), and the product can then be converted to e.g. N-(3-chloro-5-trifluoromethyl-2-pyridyl)-2,6-dinitro-3-chloro-4-trifluoromethylaniline by the method disclosed in U.S. Patent 4,331,670. The aniline derivative thus obtained shows excellent effectiveness for the control of noxious organisms, particularly of germs.

Now, the present invention will be described in further detail. However, it should be understood that the present invention is by no means restricted by these specific Examples.

EXAMPLE 1:

Into an autoclave having an internal capacity of 50 ml, 3.0 g of m-DCB, 8.0 g of carbon tetrachloride and a stirrer were put, and then cooled to 0°C. Then, 10.0 g of anhydrous hydrogen fluoride and 0.1 g of boron trichloride were added thereto. The autoclave was closed, and then the mixture was heated to 140°C and reacted for 6 hours under stirring. During the reaction, the internal pressure of the autoclave was about 71 kg/cm². After the completion of the reaction, the autoclave was cooled, and the residual pressure was gradually removed. Then, the product was poured into ice water. Then, a 20% sodium hydroxide aqueous solution was added to bring the solution alkaline, and the alkaline solution was extracted with carbon tetrachloride. The extracted layer was washed with water, dried, and then analyzed by gas chromatography, whereby the product was found to contain 88.3% of 2,4-DCBTF, 2.5% of m-DCB and 9.2% of others. This product was analyzed by an internal standard method, whereby the yield was found to be 82.2%.

EXAMPLES 2 to 10:

In the same manner as in Example 1, the respective reactions were conducted under the conditions shown in Table 1, whereby the results as shown in Table 1 were obtained.

Table 1

| Example No. | Reaction conditions | | | | | | | Yield (%) 1: 2,4-DCBTF 2: m-DCB 3: others |
| | Amount of m-DCB (g) | Amount of CCl$_4$ (g) | Amount of HF (g) | Type and amount of the catalyst (g) | Temp. (°C) | Time (hr) | Internal pressure of the auto-clave (kg/cm$^2$) | |
|---|---|---|---|---|---|---|---|---|
| 2 | 1.5 | 4.0 | 5.2 | SbCl$_5$    0.3 | 120 | 4 | 38 | 1: 76.2<br>2:  1.8<br>3: 21 |
| 3 | 1.5 | 4.0 | 5.2 | TiCl$_4$    0.2 | 140 | 18 | 48 | 1: 80.6<br>2:  3.5<br>3: 15.8 |
| 4 | 1.5 | 4.0 | 5.2 | BF$_3$    0.07 (pressurized to 0.5 kg/cm$^2$ ) | 140 | 5 | 52 | 1: 76.7<br>2:  –<br>3: 23.2 |
| 5 | 1.5 | 4.0 | 5.2 | SbCl$_5$    0.3 | 100-110 | 4 | 40 | 1: 81.6<br>2:  0.6<br>3: 17.7 |
| 6 | 3.0 | 8.0 | 5.2 | SbCl$_5$    0.3 | 120 | 4 | 82 | 1: 69.7<br>2: 14.4<br>3: 15.8 |
| 7 | 1.5 | 6.1 | 5.2 | SbCl$_5$    0.3 | 120 | 4 | 70 | 1: 79.4<br>2:  3.5<br>3: 17.0 |
| 8 | 1.5 | 4.0 | 5.2 | SbCl$_5$    0.3 | 140 | 18 | 49 | 1: 82.1<br>2:  0.6<br>3: 17.2 |
| 9 | 1.5 | 4.0 | 5.2 | NbCl$_5$    0.3 | 140 | 17 | 42 | 1: 62.2<br>2: 25.1<br>3: 12.6 |
| 10 | 1.5 | 4.0 | 5.2 | TaCl$_5$    0.36 | 140 | 14.5 | 45 | 1: 75.9<br>2:  5.9<br>3: 18.1 |
| Comparative Example | 1.5 | 4.0 | 8.4 | – | 140 | 18 | 30 | 1: 19.9<br>2: 79.4<br>3:  0.6 |

EXAMPLE 11:

Into a stainless steel autoclave equipped with a stirrer and having an internal capacity of 200 ml, 32.3 g of m-DCB, 37.3 g of carbon tetrachloride and 110 g of anhydrous hydrogen fluoride were fed, and after introducing boron trifluoride gas (0.75 g) to bring the pressure to 4.5 kg/cm$^2$, the autoclave was closed. The mixture was heated to 160$^{\circ}$C and reacted for 4 hours under stirring. During the reaction, the internal pressure of the autoclave was controlled to be at a level of 100 kg/cm$^2$, while the pressure tended to exceed this level. After the completion of the reaction, the autoclave was cooled, and the residual pressure was gradually removed. The reaction mixture was heated to from 50 to 60$^{\circ}$C to recover an excess amount of anhydrous hydrogen fluoride. The reaction product was taken out from the autoclave and analyzed by gas chromatography, whereby the product was found to contain 91.2% of 2,4-DCBTF, 1% of m-DCB and 7.8% of others. The product was neutralized with a 20% sodium hydroxide aqueous solution, and then subjected to steam distillation, whereby 37.9 g (yield: 78.8%, purity: 98.3%) of 2,4-DCBTF was obtained.

EXAMPLE 12:

Into the same autoclave as used in Example 11, 26.5 g of m-DCB, 64.4 g of trichlorofluoromethane and 90 g of anhydrous hydrogen fluoride were fed, and after introducing boron trifluoride gas (0.6 g) to bring the pressure to 3.2 kg/cm$^2$, the autoclave was closed.  The mixture was heated to 140$^{\circ}$C and reacted for 4 hours under stirring.  During the reaction, the internal pressure of autoclave was adjusted to a level of 50 kg/cm$^2$.  After the completion of the reaction, the product taken out of the autoclave was analyzed by gas chromatography in the same manner as in Example 11, whereby the product was found to contain 90.1% of 2,4-DCBTF, 0.4% of m-DCB and 9.5% of others.  The product was treated in the same manner as in Example 11, whereby 31.6 g (yield: 81%, purity: 99.2%) of 2,4-DCBTF was obtained.

CLAIMS:

1. A process for producing 2,4-dicnlorobenzotrifluoride, which comprises reacting m-dichlorobenzene with a halogenated methane of the formula $CF_xCl_{4-x}$ where x is 0, 1 or 2, and hydrogen fluoride at a temperature of from 80 to 180$^{\circ}$C in the presence of a catalyst of at least one member selected from the group consisting of fluorides of antimony, boron, titanium, tantalum and niobium.

2. The process according to Claim 1, wherein the catalyst is at least one member selected from the group consisting of fluorides of antimony, boron, titanium and tantalum.

3. The process according to Claim 1, wherein the catalyst is at least one member selected from the group consisting of fluorides of antimony, boron and titanium.

4. The process according to Claim 1, wherein the catalyst is used in an amount of from 0.02 to 10 mols per mol of m-dichlorobenzene.

5. The process according to Claim 1, wherein the catalyst is used in an amount of from 0.02 to 1 mol per mol of m-dichlorobenzene.

6. The process according to Claim 1, wherein the catalyst is used in an amount of from 0.03 to 0.3 mol per mol of m-dichlorobenzene.

7.    The process according to Claim 1, wherein the reaction temperature is from 80 to 160$^{o}$C, preferably 100 to 15o$^{o}$C.

8.    The process according to Claim 1, wherein the halogenated methane is carbon tetrachloride or trichlorofluoromethane.

9.    The process according to Claim 1, wherein the halogenated methane is used in an amount of from 1 to 10, preferably 1 to 3 mols per mol of m-dichlorobenzene.

10.    The process according to Claim 1, wherein the hydrogen fluoride is used in an amount of from 3 to 60 mols, preferably 9 to 60 mols and especially 9 to 30 mols per mol of m-dichlorobenzene.